# EUROPEAN PATENT APPLICATION

(11) **EP 1 214 946 A2**
(43) Date of publication of application: **19.06.2002**
(21) Application number: 01204798.1
(22) Date of filing: 12.12.2001
(51) Int. Cl.: A61K 49/00, A61P 35/00

(54) **Compositions and method for MRI-imaging malignant tumors using carbon 13**

(30) Priority: 13.12.2000 US 736526
(71) Applicant: Archimedes Technology Group, Inc., San Diego, CA 92121 (US)
(72) Inventor: Ohkawa, Tihiro, La Jolla, California 92037 (US)
(74) Representative: Gates, Marie Christina Esther

(57) **Abstract**

A system, method and device is described for monitoring and billing the distribution of controlled content such as copyright material over a network or set of networks. A tag is described which may be incorporated in content for distribution on media including magnetic and optical disks and electronic files accessible via a network. The tag is retained within the content and permits a network device to identify the content as it passes through the network.

## Description

The present invention relates to the distribution of content over networks, particularly although not exclusively content having copyright therein.

The distribution of content over a network or networks is a matter of prime concern to those holding rights associated with such content. The relative ease with which content of a digital character can be copied and in particular forwarded over a network to a multiplicity of recipients severely impacts the licensing potential for such content.

Hitherto, various proposals have been made to counter the loss in revenue to right holders. Broadly, these have constituted, on the one hand, the creation of technical barriers to unauthorised copying of content and on the other hand, the development of transactional controls. In many cases both approaches have been used in tandem to strictly control the distribution of content to authorised recipients namely those users who have paid the appropriate fee to the right holder.

In order to prevent unauthorised attempts to obtain the content, it has been found necessary to develop techniques that are ever more sophisticated. Consequently, the processing demands of the equipment used to generate, distribute and receive such content have inevitably grown. At the same time, the complexity and range of networks over which content may be distributed has changed such that a variety of different networks, protocols and applications may be employed.

Commercially, the impact of the above developments has been to dissuade right holders from making the investment necessary to allow access to their content such as video and audio recordings, for example.

Thus, according to one aspect of the present invention, there is provided a network device for connection in a communication path of a network includes a controller operable to detect a predetermined tag within content passing along the path and to report said detection together with information identifying a sender and/or recipient of said content.

The device may be integrated with existing equipment or provided as a separate element of the particular network architecture. Conveniently, the device could also provide routing functions in a packet switched network such as TCP/IP or ATM. The device may be utilised in predominantly circuit switched networks such as second generation Public Land Mobile Networks (PLMN) as exemplified by GSM. In these networks, so-called short messages of a textual and/or audio nature may be delivered between terminals as datagrams. Such messages pass through a short messaging centre which may incorporate the device. Consequently, datagrams comprising content with which digital rights are associated may be detected.

According to another aspect of the present invention, a method of monitoring content transmitted over a network comprises detecting a predetermined tag within content passing through the network and reporting said detection together with information identifying a sender and/or recipient of said content.

Preferably, the tag is associated with the content before it is made available for transmission over the network. Thus the tag, which may be suitably encrypted and/or concealed may take the form of a watermark, flag in the header or otherwise embedded in the content. As an alternative or adjunct to encrypting the tag, the transmission itself may be encrypted. In addition, the content may be generated to include spoiler code to prevent its playback, for example, if the tag is removed.

The tag is associated with the content by a right holder or a party authorised by him, during the preparation of the content for distribution by any suitable channel. The content and tag could be carried on any suitable data carrier such as a Compact Disc (CD) Digital Versatile Disc (DVD) or other means of distribution including making it available for download for a fee from a website. As a result, any subsequent dealings with the content such as uploading it to a network for distribution as an MP3 file for example would result in the tag being available for detection.

According to a further aspect of the present invention, there is provided a system for monitoring the transmission of content between networked terminals comprises, a network device located in a communication path of a network and a monitoring centre connected to said device wherein a controller included in said device is operable to detect a predetermined tag within content in said path and to report said detection together with information identifying a sender and/or recipient of said content to said centre.

Preferably, the device is placed in a communication path through which substantially all traffic carried by that network passes. Clearly, where network conditions dictate, then a number of such devices may be utilised in different parts of the network to carry respective portions of the traffic. Where more than one network is involved in the transmission of the content then each device may selectably report information relating to a sender or recipient of said content. Consequently, the number of reports generated is kept to a minimum and in addition multiple reporting of content as it passes through the or each network is avoided. This results in reduced network load and simplifies any resultant action taken as a result of such reporting including billing the sender or recipient for the transmission of said content.

According to a still further aspect of the present invention, there is provided a revenue collection system for collecting revenue due on content passing through a network, comprises a network device located in a communication path of said network and a monitoring centre connected to said network device wherein said network device is operable to detect a predetermined tag within content in said path and to report said detection together with information identifying a sender and/or recipient of said content to said centre, the centre being operable to issue a request to a billing entity to carry out a transaction in relation to said sender and/or recipient.

The monitoring centre may be integrated with the device such that both fall under the control of a party responsible for the network in which they are located. Such a scenario provides benefits to the operator of a PLMN or ISP who could integrate the billing for transfer of such content with his normal operations whilst keeping the information generated thereby within the confines of his network. However, in some circumstances, perhaps where a copyright collection authority wishes not only to carry out billing but also to monitor usage, then the centre could be located remotely of the device perhaps in a separate network to which the device addresses its reports.

According to yet another aspect of the present invention, there is provided a revenue collection method for collecting revenue due on predetermined content transmitted over a network comprises detecting said predetermined content, obtaining an address of a sender and/or recipient of said content and requesting a billing entity responsible for said sender and/or recipient to debit their corresponding account.

The invention provides an open solution to the problem of digital rights management of copyright content in particular. Content may be received in a terminal from an external source and then passed to another terminal where the content can accessed without any effect on the embedded tag. Such an open solution provides immense benefits in that it may be applied to existing technology without the need for modification of terminals, in particular. Hitherto, as has previously stated, digital rights management requires the presence of encryption, decryption and digital right management software on the terminal. Furthermore, the invention is applicable to any network or combination of networks where billing structures exist or could be implemented to bill usage of terminals connected to that network. Thus, PC usage could be billed by a respective ISP and mobile terminal usage could be billed by a respective network operator. Clearly, the level of information reported for the purposes of billing or the like could be increased from a minimum namely that the material is controlled in the sense that it may be copyright to add details of the distributor, record company and artiste in the case of a musical work, for example. Such additional rights management information is particular useful in establishing a revenue distribution arrangement for right holders.

It will also be desirable to provide a terminal user with the option to abort any attempt he might make to forward controlled content which, by implication could involve him in being billed. The terminal user will be asked to confirm his request to send controlled content together with an indication of the expected cost. Such a confirmation request could be responded to simply by selecting send or reject as actions via the terminal user interface. Most conveniently, the option could be generated on the network side in response to a request received from the terminal to send controlled content. This would avoid any need for a terminal implementation. However, the terminal could be provided with the resources necessary to identify controlled content before sending the content onto the network. Advantageously, there could further be provided a mechanism whereby on identification of such content the user is given the option to send the content once a predetermined billing activity has successfully been completed over the network with a credit card company or the like.

In order to understand the present invention more fully, a number of embodiments thereof will now be described by way of example and with reference to the accompanying drawings, in which:
Figure 1 is a diagrammatic view of a network portion of a content distribution monitoring system according to one embodiment of the present invention;
Figure 2 is a similar view of a management portion of the content distribution system of Figure 1;
Figure 3 is a diagrammatic view of a further embodiment of the system of Figure 1;
Figure 4 is a diagrammatic view of one example of the system of Figure 2 in use.
Figure 5 is a diagrammatic view of an example of the system of Figure 1 in use;
Figure 6, is a chart illustrating the generation of content for use with the system of Figures 1,2 and 3.

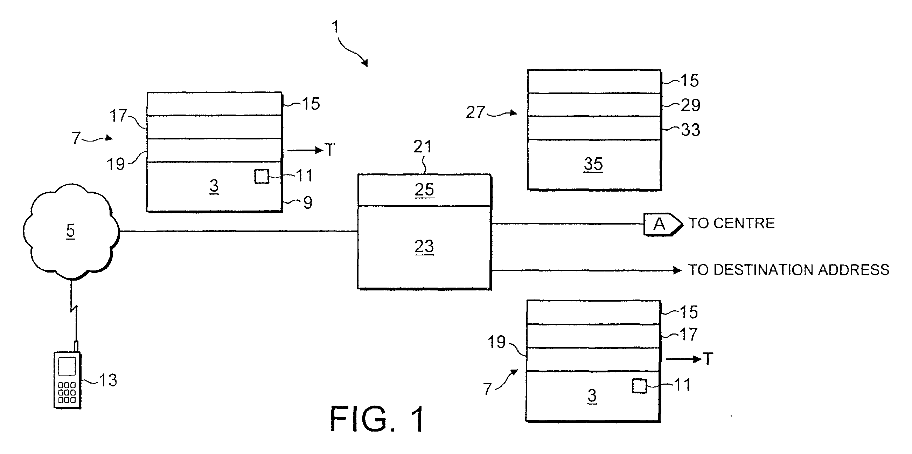

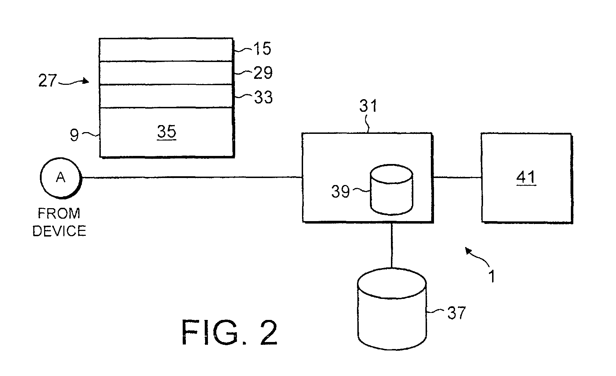

Referring to Figures 1 and 2 in particular, there is shown an embodiment of a system 1 for monitoring the distribution of content 3 over a network 5. Content 3 is encapsulated in a datagram 7 formatted in accordance with the frame type of the protocol the network 5 is operating. The datagram 7 is understood to be travelling through the network in a path indicated by an arrow marked T. The content 3 is located in a payload portion 9 of the datagram 7. Where monitoring of the content 3 is required, such as for the management of copyright and the like, a marker or tag 11 is stored together with the content 3 in the datagram 7. The introduction of the tag 11 may be carried out by software during the creation of the content 3 or indeed at a later stage either before or during generation of the datagram 7 by a device 13 able to communicate with the network 5. In addition to the payload portion 9 containing the content 3, the datagram 7 is provided with fields containing a destination address 15 and respectively the address 17 of the sender's device 13 and an initial router address 19 determined from a look-up table or similar held by the sender's device 13.

A network device 21 operated by a provider responsible for operating the network 5 in which the device 21 resides includes a filtering router 23 and controller 25. On reaching the router 23 the datagram 7 is examined and the destination address 17 is compared against a table held by the router 23 with a view to forwarding the datagram 7 to either the destination address 15 or to another router (not shown) perhaps on another network which contains the destination address 15. In the latter case, the router address 19 will reflect the location of the new router, otherwise it will correspond to the destination address. In the meantime, the controller 25 firstly examines the sender's address 15 of the datagram 7 to determine whether it originates from a device located in the same network as the controller 25 if so, the application further examines the location within the datagram 7 at which the tag 11 may be located. The exact location of the tag 11 may be concealed whilst the tag 11 itself may be encrypted. A controller application is therefore arranged to locate the tag 11 and decrypt any protection previously applied thereto. If the tag 11 is present and indicates the presence of controlled content such as copyright or classified material, for example, then the application generates an additional control datagram 27 which contains the address 15 of the originating device which is once is again stored in the originating address field. In addition, a destination address 29 is entered corresponding to the address of a monitoring centre 31 and a routing address 33 is also entered which may or may not correspond to the destination address 29 depending on whether the monitoring centre 31 can be seen by the filtering router 23. Indeed the centre 31 may be located on a different network in which case the control datagram will follow an appropriate routing to reach the centre 31 (shown in the figure by the indicia A). The controller 25 application also places a flag 35 in the payload portion 9 of the control datagram 27 to identify to the centre 31 in due course that controlled content has passed through that device 21. Alternatively, rather than have the controller 25 place a flag in the control datagram 27, the centre 31 may identify the access point into the network 5 by reference to the originating address 15.

The centre 31 receives the datagram 27 generated in the device 21 and is able to establish from the originating address 15 and the flag 35 in the payload portion 9 that controlled material has been sent by the sender's device 13. The centre 31 then contacts a billing entity 37 responsible for charging for access to the home network, in this case the network 5 in which the device 21 is located, of the sender's device 13 with an instruction to debit an account belonging to a user of the device 13 by a predetermined sum. The billing entity 37 responds to the request by crediting a revenue account held by the operator of the centre 31 with the sum and logs the debit on the user's account record. In the meantime, the centre 31 logs the receipt of the datagram 27 in a memory 39 together with the flag data 35.

Subsequently, parties such as right holders having an interest in the controlled content 3 are provided with an apportionment of the sums deposited in the revenue account. The precise apportionment of the sums to each party may be established by separate negotiation and/or through an analysis of the log held by the centre via a link to a collection authority server 41. Thus, in one variant of the embodiment, the flag 35 contains additional indicia identifying one if not all of the following, namely the copyright holder, distributor, record company, artist, additional or alternative fields may, of course be included for other non-musical works, for example. It will also be apparent that the centre 31 may be independent of the control of the network operator responsible for the network device 21. Accordingly, some portion of the revenue collected by the centre 31 may be apportioned to the operator of the device 21. Clearly, where more than one such device 21 each located in a different home network is responsible for forwarding datagrams to the centre 31, the apportionment may vary depending on the level of content transmitted by users of each home network. Again, this apportionment could be determined by separate agreement or more likely through examination of the log in memory 39 to reveal the owner of the network device 21 responsible for forwarding the control datagram 27.

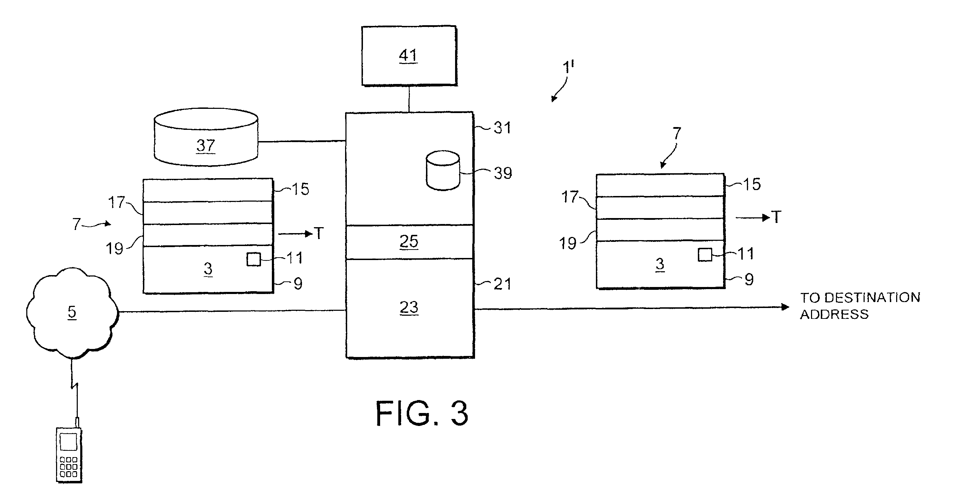

Figure 3 shows another embodiment of a system for system for monitoring the distribution of content over a network. As previously stated in relation to the earlier embodiment, content is encapsulated in a datagram formatted in accordance with the frame type of the protocol the network is operating.

Indeed, in all other respects the datagram is no different to that previously described and the same reference numerals will be utilised where appropriate.

In this further embodiment, the monitoring centre 31 is integrated with the network device 21. Consequently, the network operator responsible for the network device 21 has authority over the system 1'. Thus, rather than the controller 25 application generate an additional datagram, the information necessary to generate revenue for the right holders is extracted by the application and passed directly to the centre 31. Although the combination of the network device 21 and centre 31 could be arranged to receive control datagrams from external networks, this would necessitate the provision of a link to the billing entities of those external networks. Otherwise, it is only necessary for the centre to have a link to the billing entity 37 of its own network and, of course, provision for right holders to access the log held in memory 39.

In use, content 3, the transmission of which should result in revenue being generated for the appropriate right holder, may enter, leave and transit a network or set of networks in a number of ways. The following examples illustrate at least some of the potential scenarios.

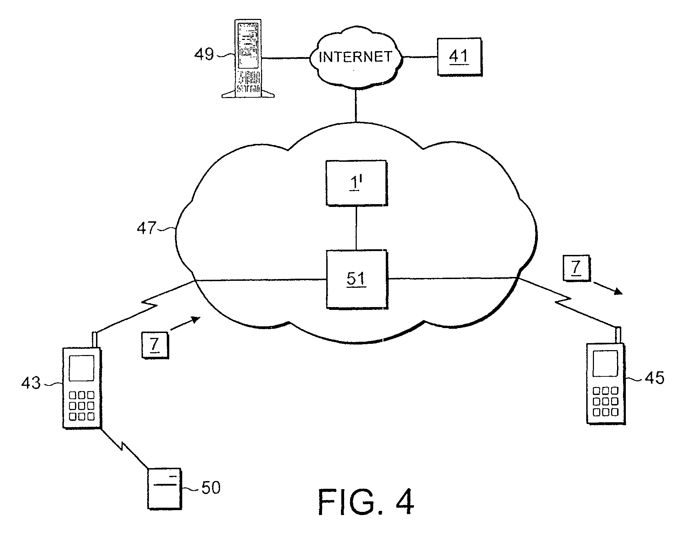

Thus, Figure 4 illustrates the transfer of controlled content from a first terminal 43 to a second terminal 45, both of which are connectable to a common network, namely a Public Land Mobile Network (PLMN) 47. The controlled content to be sent from the first terminal may have been previously downloaded from a distribution point such as a website 49 of a right holder or uploaded from another device such as an MP3 player 50 or the like connected to the first terminal 43. In order to forward the content, the device 43 places the content into a datagram 7 which, in the manner well known to the art, is passed over the network 47 to the recipient terminal 45 via a messaging centre 51, once a preliminary step of confirming the transfer and billing amount has been completed. Connected to the messaging centre 51 is a combined network device 21 and monitoring centre 31 corresponding to the further embodiment 1' of the invention. Subsequently, the sender's address is derived from the datagram 7, which in the case of a GSM network could be a short message (SM), and is used by the billing entity of the network 47 to charge the account corresponding to the first (sending) terminal. Thus, the sender's address may be a telephone number or IP address of the first terminal. In addition, as has already been described, a corresponding entry is made in the centre log which may subsequently accessed by the right holder via the collection body 41.

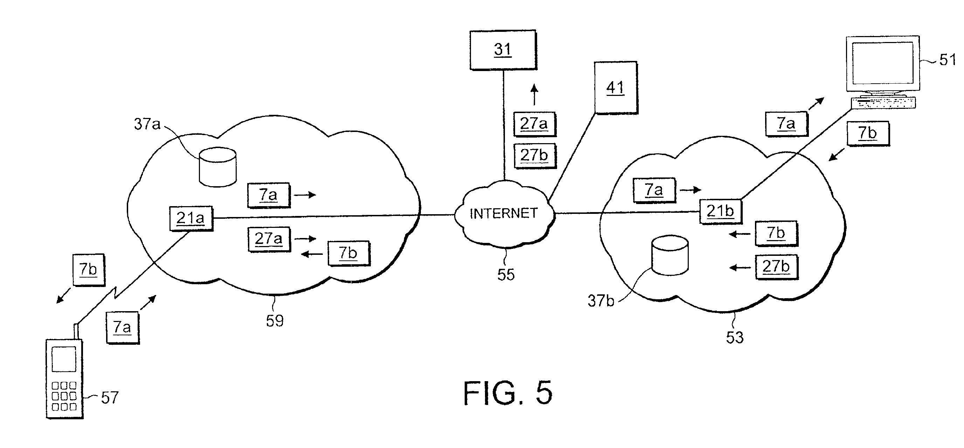

Figure 5 illustrates a transfer of controlled content between a Personal Computer (PC) 51 connectable via an Internet Service Provider (ISP) 53 to a global information network such as the Internet 55 and a mobile terminal 57 connectable to a PLMN 59. The PLMN 59 is in turn provided with a connection to the Internet 55. Each provider, namely the ISP and the PLMN operator include a network device 21a, 21b within its respective network architecture 53,59. In this example, each device 21a,21b is linked to an independently operated monitoring centre 31. Thus, when a datagram 7a containing controlled content 3 is generated at the mobile terminal 57, its progress towards the PC 51 is detected by both devices 21a, 21b. However, only the device 21 a in the home network 59 of the mobile terminal 57, namely the PLMN 59 reports the fact by generating an additional datagram 27a addressed to the centre 31, as it recognises the sending address of the content encapsulating datagram 7a to be within its own network 59. Similarly, when a datagram 7b originating in the PC 51 is sent to the mobile terminal 57, only the device 21b in the ISP architecture 53 reports the fact with a control datagram 27b addressed to the centre 31. In a non-illustrated variant of this example, one or both devices 21a,21b are integrated with a monitoring centre 31 under the control of the respective home network 59,53. As was explained previously, this still results in the appropriate revenue being generated for the right holders.

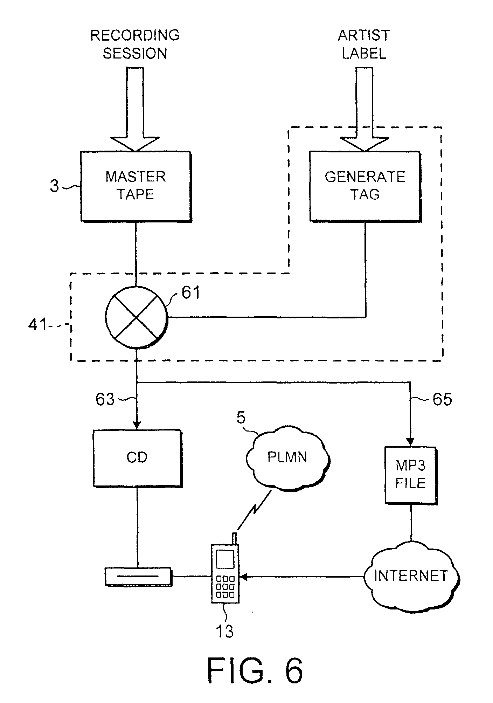

Figure 6 illustrates a service through which a tag 11 may be added to a content 3 to permit control thereof. A party authorised by the right holder prepares a master file of content 3 for ultimate distribution and forwards the content on a data carrier or electronically over a communication network or using a data carrier to a copyright collection body 41 or the like. The collection body receives the content and identifies the right holder from identity data provided by the right holder such as artist and label, for example. The collection authority may derive the right holder identity in the case of the electronically communicated content from a sender's address field. The collection body 41 then updates its record of copyright content in the name of the right holder and associates 61 the content 3 with the tag 11. Such information may be used in determining the level of apportionment of revenue to the right holder subsequently. The tagged content 3 may then be distributed by a suitable channel 63,65 to a user who is then free to upload the content to his home network 5 using his terminal 13.

Thus, content may be distributed initially on a data carrier such as a Compact Disk (CD) a Digital Versatile Disk (DVD) or memory stick, for example. The content may also be distributed electronically by offering it for download at a website, for example.

In a further non-illustrated variant a right holder or another authorised party may obtain the resources which might include software and/or hardware to place a tag on the content. The content may then be distributed as has been outlined previously. Clearly, such an approach may require separate notification to the collection body of the generation of new controlled content. This will particularly be the case where revenue apportionment is based on the amount of content of a right holder in circulation.

## Claims

1. A network device for connection in a communication path of a network includes a controller operable to detect a predetermined tag within content passing along the path and to report said detection together with information identifying a sender and/or recipient of said content.

2. A method of monitoring content transmitted over a network comprises detecting a predetermined tag within content passing through the network and reporting said detection together with information identifying a sender and/or recipient of said content.

3. A computer program product for carrying out the method according to claim 2 comprising: a computer readable medium;
program code in said computer readable medium for detecting a predetermined tag within a content passing through a network;
program code in said computer readable medium for reporting said detection together with information identifying at least one of the following: a sender of said content, a recipient of said content.

4. A system for monitoring the transmission of content between networked terminals comprises, a network device located in a communication path of a network and a monitoring centre connected to said device wherein a controller included in said device is operable to detect a predetermined tag within content in said path and to report said detection together with information identifying a sender and/or recipient of said content to said centre.

5. A system as claimed in Claim 4, wherein the monitoring centre is operable to receive reports from a plurality of networks each having at least one controller.

6. A system as claimed in Claim 4 or Claim 5, further including a billing entity connected to said centre.

7. A revenue collection system for collecting revenue due on content passing through a network, comprises a network device located in a communication path of said network and a monitoring centre connected to said network device wherein said network device is operable to detect a predetermined tag within content in said path and to report said detection together with information identifying a sender and/or recipient of said content to said centre, the centre being operable to issue a request to a billing entity to carry out a transaction in relation to said sender and/or recipient.

8. A system as claimed in Claim 7 wherein the transaction comprises debiting an account of said sender and/or recipient.

9. A system as claimed in Claim 7 or Claim 8, wherein said information includes a network identity such that said centre issues said request to a billing entity responsible for said identified network.

10. A revenue collection method for collecting revenue due on predetermined content transmitted over a network comprises detecting said predetermined content, obtaining an address of a sender and/or recipient of said content and requesting a billing entity to carry out a transaction in relation to said sender and/or recipient.

11. A method as claimed in Claim 10, wherein said transaction comprises debiting an account of said sender and/or recipient

12. A method as claimed in Claim 10 or Claim 11, including the preliminary step of determining which content revenue is to be collected by associating a tag with said content prior to making it available for transmission.

13. A method as claimed in Claim 12, wherein said predetermined content is captured on a data carrier.

14. A method of transmitting a message incorporating content including an embedded tag from a terminal connected to a network comprises, obtaining content, placing said content into a payload portion of said message, and transmitting said message over a network including a device as claimed in Claim 1.

15. A method as claimed in Claim 14, wherein the content is obtained by downloading from a server.

16. A method as claimed in Claim 14, wherein the content is obtained from a data carrier by uploading from a suitable player.

17. A method as claimed in any one of Claim 14 to 16, wherein said message comprises one or more packets.

18. A method as claimed in claim 17, wherein said tag is embedded to at least one of the packets

19. A method of creating content for controlled distribution over a network comprises, generating content, determining a right holder and updating a corresponding right holder record with details of said content and associating a tag with said content wherein said tag is detectable by a device as claimed in Claim 1.

20. A computer program comprising executable code for execution when loaded on a computer, wherein the computer is operable in accordance with said code to carry out the method according to Claim 2.

21. A computer program comprising executable code for execution when loaded on a computer, wherein the computer is operable in accordance with said code to carry out the method according to any one of Claims 10 to 13.

22. A computer program comprising executable code for execution when loaded on a computer, wherein the computer is operable in accordance with said code to carry out the method according to any one of Claims 14 to 17.

23. A computer program comprising executable code for execution when loaded on a computer, wherein the computer is operable in accordance with said code to carry out the method according to Claim 19.

24. A program as claimed in any one of Claims 20 to 23, stored in a computer readable medium.

25. A method of generating a control message by a network device to be sent to a monitoring centre connected to the said device, the message indicating the passing of a content having a predetermined tag embedded within the content through the network device, the control message comprising an identification of the content originating device, a destination address for the content, and a flag created by the network device.

26. A method as claimed in Claim 25, wherein the flag identifies the network device.
